# EUROPÄISCHE PATENTSCHRIFT

(11) **EP 0 552 203 B1**
(45) Veröffentlichungstag und Bekanntmachung des Hinweises auf die Patenterteilung: **03.05.1995**
(21) Anmeldenummer: 91917451.6
(22) Anmeldetag: 04.10.1991
(51) Int. Cl.: C12Q 1/68

(54) **VERFAHREN ZUM EMPFINDLICHEN NACHWEIS VON NUKLEINSÄUREN**
NUCLEIC ACID SENSITIVE DETECTION PROCESS
PROCEDE DE DEPISTAGE SENSIBLE D'ACIDES NUCLEIQUES

(30) Priorität: 09.10.1990 DE 4032024; 05.12.1990 DE 4038804; 22.12.1990 DE 4041608
(43) Veröffentlichungstag der Anmeldung: 28.07.1993
(73) Patentinhaber: BOEHRINGER MANNHEIM GMBH, 68298 Mannheim (DE)
(72) Erfinder: KESSLER, Christoph, D-8021 Dorfen (DE); RÜGER, Rüdiger, D-8124 Seeshaupt (DE); SEIBL, Rudolf, D-8122 Penzberg (DE); KRUSE-MÜLLER, Cornelia, D-8132 Tutzing (DE); BERNER, Sibylle, D-8900 Augsburg 1 (DE)
(74) Vertreter: Kolb, Bernd, Dr.
(86) Internationale Anmeldenummer: EP9101898
(87) Internationale Veröffentlichungsnummer: WO9206216

(56) Entgegenhaltungen:
- EP-A- 0 329 822
- EP-A- 0 395 398
- WO-A-90/11374
- AT-B- 390 080
- GB-A- 2 202 328
- PROCEEDINGS OF THE NATL. ACADEMY OF SCIENCES USA, Band 86, August 1989, Washington, DC (US); R. K. SAIKI et al., Seiten 6230-6234#

## Beschreibung

Gegenstand der Patentanmeldung ist ein Verfahren zum spezifischen Nachweis einer Nukleinsäure oder eines Teils davon.

Der Nachweis von Nukleinsäuren in der klinischen Diagnostik und der Molekularbiologie hat in jüngster Zeit gegenüber den klassischen Immuntests immer mehr an Bedeutung gewonnen. Dies hängt damit zusammen, daß Fortschritte im Hinblick auf die Erforschung der Nukleotid-Sequenz von Nukleinsäuren verschiedenen Ursprungs gemacht wurden.

Da die in der Nukleotidsequenz enthaltenen Informationen sehr differenziert sind, kann die Nukleinsäurediagnostik besonders vorteilhaft zur Unterscheidung geringster Merkmale eingesetzt werden. Dies ist beispielsweise wichtig beim Nachweis von Mutationen und Unterschieden in künstlich erzeugten Sequenzen. Durch die Einführung von Amplifizierungsverfahren konnte die Empfindlichkeit von Nukleinsäurenachweisen erheblich gesteigert werden.

Ein solches Verfahren ist beispielsweise in der EP-A-0 200 362 beschrieben. Der Amplifikationseffekt beruht im wesentlichen darauf, daß aus einer sogenannten target-Nukleinsäure als template mit Hilfe eines Primers und Mononukleosidtriphosphaten ein Verlängerungsprodukt des Primers gebildet wird, das entweder nachgewiesen wird oder selbst wieder als template-Nukleinsäure verwendet werden kann. Dabei kann in das Verlängerungsprodukt auch ein nachweisbares Nukleotid eingebaut werden. Das entstandene Verlängerungsprodukt kann elektrophoretisch nachgewiesen werden.

Diese Nachweismethode ist jedoch wegen des umständlichen und zeitaufwendigen Elektrophoreseschritts nachteilig.

Das nicht markierte Verlängerungsprodukt kann gemäß EP-A-0 201 184 auch durch Hybridisierung mit einer nachweisbar markierten Nukleinsäuresonde nachgewiesen werden. Die Abtrennung des Hybrids aus Verlängerungsprodukt und Sonde von unumgesetzter Sonde ist jedoch mit dem dort beschriebenen Verfahren wegen unspezifischer Wechselwirkungen entweder ineffizient oder mit vielen Waschschritten verbunden, welche die Empfindlichkeit herabsetzen.

In der WO 89/11546 wird ein Verfahren vorgeschlagen, bei dem amplifizierte, festphasengebundene Nukleinsäuren mit einem Primer und markierten Mononukleotiden umgesetzt werden, wobei die dabei gebildeten markierten Nukleinsäuren nachgewiesen werden. Dieses Verfahren hat den Nachteil, daß alle maßgeblichen Reaktionen an der festen Phase ablaufen, wodurch die Reaktionsgeschwindigkeit beeinträchtigt ist. Auch in der EP-A-0 324 474 wird ein solches Verfahren vorgeschlagen, bei dem markierte Mononukleotide eingebaut und diese markierten Nukleinsäuren mit zur nachzuweisenden Nukleinsäure komplementären immobilisierten Nukleinsäuren abgefangen werden. Über die Methode der Denaturierung der amplifizierten Nukleinsäuren und die Hybridisierungsbedingungen ist nichts ausgesagt. Ein weiterer Nachteil dieses Verfahrens ist, daß die Herstellung effizient festphasengebundener Nukleinsäuren aufwendig ist.

In der EP-A-0 357 011 wird ein Verfahren beschrieben, bei dem in die Verlängerungsreaktion zwei Primer eingesetzt werden, von denen einer nachweisbar markiert und der andere zur Bindung an eine feste Phase geeignet ist. Dieses Verfahren hat den Nachteil, daß es aufwendiger ist, nachweisbar markierte Oligonukleotide von den diese Oligonukleotide beinhaltenden Verlängerungsprodukten abzutrennen. Wenn keine Abtrennung vorgenommen wird, ist wegen Konkurrenzreaktionen eine verringerte Sensitivität zu erwarten.

In der EP-A-0 297 379 und der EP-A-0 348 529 ist ein Verfahren beschrieben, bei dem ein immobilisierter oder immobilisierbarer Primer unter Zuhilfenahme der target-Nukleinsäure als template mit einem detektierbaren Mononukleotid zu einer immobilisierten und gleichzeitig nachweisbar markierten Nukleinsäure verlängert wird. Dieses Verfahren hat u.a. den Nachteil, daS die Spezifität des Nachweises nicht sehr hoch ist. Das ebenfalls in EP-A-0 297 379 beschriebene Verfahren, bei dem nur ein immobilisierter oder immobilisierbarer Primer eingesetzt wird, woraufhin die Verlängerungsprodukte mit einem markierten Oligonukleotid umgesetzt werden, hat den bei EP-A-0 357 011 beschriebenen Nachteil der erschwerten Abtrennbarkeit des Oligonukleotids.

In der WO 89/09281 ist ein Verfahren beschrieben, bei dem die beiden Primer dieselbe chemische Gruppe aufweisen, die einmal zur Immobilisierung und zum anderen zum Nachweis verwendet wird. Dies verstärkt den oben genannten Nachteil der schlechten Abtrennbarkeit noch.

In Proc. Natl. Acad. Sci. USA, Vol. 86, pp 6230-6234 wird ebenfalls ein Verfahren geschildert, in dem ein nachweisbar markierter Primer verlängert wird. Der Nachweis erfolgt nach Bindung der Verlängerungsprodukte an eine Fangprobe. Auch hier ist die Abtrennung des nachweisbar markierten Primers nicht ohne Zusatzschritte möglich, und er stört daher die Nachweisreaktion.

Es war daher Aufgabe der Erfindung, die Nachteile des Verfahrens des Standes der Technik zu vermeiden und insbesondere ein Nukleinsäurenachweisverfahren bereitzustellen, welches hohe Spezifität oder Selektivität mit geringem Hintergrundsignal verbindet.

Gegenstand der Erfindung ist ein Verfahren zum spezifischen Nachweis einer Nukleinsäure in einer Probe, welches folgende Schritte umfaßt:
a) Reaktion der Probe mit einem oder mehreren mit einer nachweisbaren Gruppe markierten Mononukleosidtriphosphaten und einem oder mehreren Enzymen, welche die Herstellung einer markierten Nukleinsäure B katalysieren, die dieses Nukleotid enthält,
b) nichtthermische Denaturierung der Reaktionsmischung,
c) Hybridisierung der Nukleinsäure B mit einer Nukleinsäuresonde C, welche mindestens eine immobilisierbare Gruppe enthält und welche hinreichend komplementär zu der Nukleinsäure B ist, und gleichzeitige Immobilisierung der Nukleinsäuresonde C an eine feste Phase, welche die immobilisierbare Nukleinsäuresonde C spezifisch binden kann,
d) Trennung der flüssigen von der festen Phase, und
e) Nachweis des aus markierter Nukleinsäure B und Nukleinsäuresonde C gebildeten Nukleinsäurehybrids D, indem die an die feste Phase gebundene nachweisbare Gruppe nachgewiesen wird,
wobei sich die immobilisierbare Gruppe von der nachweisbaren Gruppe unterscheidet.

Bei dem erfindungsgemäßen Verfahren handelt es sich um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, 2.9.1. - 2.9.10 und Molecular Cloning, Edt. J. Sambrook et al., CSH, 1989, 9.4.7. - 9.5.8. Bezug genommen. Dazu gehören insbesondere die bekannten Methoden zur Herstellung von markierten Nukleosidtriphosphaten, wie sie auch in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungs-bedingungen, durch welche eine Spezifität erreicht werden kann, die vom Ausmaß der Homologie zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe L. Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Nukleosidtriphosphate (NTP) sind Ribo (rNTP)- oder Desoxyribonukleosidtriphosphate (dNTP).

Unter einer target-Nukleinsäure wird eine Nukleinsäure verstanden, die Ziel des Nachweises und Ausgangsstoff des erfindungsgemäßen Verfahrens ist.

Eine template-Nukleinsäure A ist eine Nukleinsäure, zu der ein im wesentlichen komplementärer Nukleinsäurestrang neu gebildet wird. In Bezug auf die Sequenzinformation dient die template-Nukleinsäure als Matritze und enthält die Sequenzformation, die in Reaktion a) in die Nukleinsäure B umgeschrieben wird. Die Nukleinsäure A ist entweder die target-Nukleinsäure oder eine davon abgeleitete Nukleinsäure. Sie kann beispielsweise ein Teil der target-Nukleinsäure sein oder einen Teil der target-Nukleinsäure neben anderen Teilen enthalten, beispielsweise hochkomplexe Nukleinsäuren. Sie kann auch einen Teil des zu der target-Nukleinsäure komplementären Stranges enthalten.

Denaturierung von Nukleinsäuren bedeutet Auftrennung von Nukleinsäuredoppelsträngen in Einzelstränge. Dem Fachmann stehen eine Vielzahl von Varianten zur Verfügung.

Unter einem spezifischen Nachweis wird ein Verfahren verstanden, durch welches gewünschtenfalls selektiv bestimmte Nukleinsäuren auch in Gegenwart anderer Nukleinsäuren nachgewiesen werden können. Es ist jedoch auch möglich, mehrere Nukleinsäuren oder eine Gruppe von Nukleinsäuren mit teilweise übereinstimmender oder ähnlicher Nukleotidsequenz oder mehrere Abschnitte einer Nukleinsäure in Gegenwart anderer Nukleinsäuren nachzuweisen. Zum Nachweis doppelsträngiger Nukleinsäuren kann jeder der beiden komplementären Stränge einbezogen werden.

Unter einer im wesentlichen komplementären Nukleinsäure oder Nukleinsäuresequenz werden Nukleinsäuren oder Sequenzen verstanden, die mit der entsprechenden Nukleinsäure hybridisieren können, deren Nukleotidsequenz im hybridisierenden Bereich entweder genau komplementär zu der anderen Nukleinsäure ist oder sich in wenigen Basen von der genau komplementären Nukleinsäure unterscheidet. Die Spezifität richtet sich dabei sowohl nach dem Grad der Komplementarität als auch nach den Hybridisierungsbedingungen.

Flüssige Phasen sind die üblicherweise bei Nukleinsäurenachweisen verwendeten wässrigen Phasen mit gelösten organischen bzw. anorganischen Inhaltsstoffen, z.B. Hybridisierungspuffer, überschüssigen Nukleotiden, weiteren nicht nachzuweisenden Nukleinsäuren, Proteinen etc.

Das erfindungsgemäße Verfahren dient zum Nachweis einer Nukleinsäure. Unter Nukleinsäuren sind hierbei Nukleinsäuren jeglichen Ursprungs zu verstehen, beispielsweise Nukleinsäuren viroiden, viralen, bakteriellen oder zellulären Ursprungs. Sie können in Lösung, Suspension, aber auch an Festkörpern fixiert oder in zellhaltigen Medien, Zellabstrichen, fixierten Zellen, Gewebsschnitten oder fixierten Organismen vorliegen. Bevorzugt liegen die Nukleinsäuren in Lösung vor. Die Reaktionssequenz wird meist gestartet durch Verfügbarmachung der zu untersuchenden Nukleinsäure mit entsprechenden Reagentien. Hierbei können sowohl Veränderungen des pHs (alkalisch), Hitze, Wiederholung extremer Temperaturveränderungen (Einfrieren/Auftauen), Veränderung der physiologischen Wachstumsbedingungen (Osmotischer Druck), Einwirkung von Detergentien, chaotropen Salzen oder Enzymen (z.B. Proteasen, Lipasen), alleine oder in Kombination zur Freisetzung der Nukleinsäuren beitragen. Da das erfindungsgemäße Verfahren sehr empfindlich und selektiv ist, können auch kleine Nukleinsäuremengen in Anwesenheit anderer Stoffe, beispielsweise Proteinen, Zellen, Zellfragmenten, aber auch nicht nachzuweisender Nukleinsäuren nachgewiesen werden. Eine Aufreinigung von Proben kann somit entfallen, wenn sichergestellt ist, daS die nachzuweisenden Nukleinsäuren für die eingesetzten Reagenzien ausreichend zugänglich sind.

Die Nukleinsäuren können auch vorbehandelt sein. Zu den bekannten Vorbehandlungen gehört insbesondere die Fragmentierung, beispielsweise mittels Restriktionsenzymen oder die cDNS-Synthese aus RNS. Damit das erfindungsgemäße Verfahren seine Vorteile voll entfalten kann, hat es sich als zweckmäßig erwiesen, wenn die Nukleinsäure eine Größe von mindestens 40 bp aufweist.

Weiterhin ist die Nukleinsäure bevorzugt das Produkt einer vorgeschalteten spezifischen oder unspezifischen Nukleinsäurevermehrung. Solche Nukleinsäureamplifikationsverfahren sind beispielsweise aus
EP-A-0 201 184, EP-A-0 272 098, DE-A-37 26 934, EP-A-0 237 362, WO 88/10315, WO 90/01069, WO 87/06270, EP-A-0 300 796, EP-A-0 310 229, WO 89/09835, EP-A-0 370 694, EP-A-0 356 021, EP-A-0 373 960, EP-A-0 379 369, WO 89/12696 oder EP-A-0 361 983
bekannt. Die Nukleinsäure kann jedoch auch eine durch Klonierung und in-vivo-Vermehrung hergestellte Nukleinsäure sein.

Die nachzuweisenden (target-)Nukleinsäuren können direkt als template-Nukleinsäuren A in Reaktion a) eingesetzt werden, wenn sie die für das gewählte Enzymsystem erforderlichen Bedingungen erfüllen. Dazu gehört für manche Enzyme, daß es sich um einzelsträngige Nukleinsäuren handelt, bei anderen ist es erforderlich, daß sie Erkennungsstellen bzw. Promotoren für das Enzymsystem beinhalten.

Ist dies nicht der Fall, so müssen die target-Nukleinsäuren in einem (Reaktion a) vorgeschalteten Schritt in derartige template-Nukleinsäuren A überführt werden.

Es kann sich bei A um eine Ribonukleinsäure oder eine Desoxyribonukleinsäure handeln. Besonders bevorzugt als Nukleinsäure A sind Desoxyribonukleinsäuren.

Das Enzym E im Sinne der Erfindung ist ein Enzym oder Enzymsystem, das die templatabhängige Synthese von Nukleinsäuren aus Mononukleosidtriphosphaten katalysiert. Bevorzugte Enzyme sind Polymerasen und Transkriptasen, die die Verknüpfung von Mononukleotiden bewirken. Solche Enzyme sind dem Fachmann bekannt.

In einem ersten Schritt wird aus der template-Nukleinsäure A eine markierte Nukleinsäure B hergestellt. Dies kann auf prinzipiell jede Art und Weise geschehen, solange nur die spezifische Information der Nukleotidsequenz der Nukleinsäure A oder eines Teils davon im wesentlichen erhalten bleibt.

Eine Methode ist beispielsweise der Austausch von einzelnen Nukleotiden der Nukleinsäure A gegen markierte Nukleotide, beispielsweise durch Nick-Reparatur (J. Mol. Biol. 113, 237 (1977)). Das Enzym E bei dieser Reaktion ist E. coli DNA Polymerase oder Kornberg-Enzym. Dieses Verfahren ist jedoch nicht allzu vorteilhaft, da die Markierung relativ unspezifisch eingeführt wird, d.h. neben der Nukleinsäure A alle weiteren in der Probe vorliegenden Nukleinsäuren markiert werden. Es eignet sich daher für Reaktion a) insbesondere dann, wenn, wie oben geschildert, in einer Vorgeschalteten Vermehrungsreaktion die nachzuweisenden Nukleinsäuren spezifisch amplifiziert wurden.

In einer anderen Methode, welche ebenfalls den genannten Nachteil aufweist, wird die Nukleinsäure A unter Einbau von markierten Nukleotiden verlängert (tailing). Weitere Verfahren dieser Art sind Photomarkierung und Random Priming.

Bei den bisher geschilderten Schritten zur Herstellung der Nukleinsäure B wird in Reaktion A direkt die target-Nukleinsäure eingesetzt. Diese kann einzel- oder doppelsträngig vorliegen.

Die target-Nukleinsäuren der im folgenden aufgeführten bevorzugten Ausführungsvarianten müssen vor oder während der Reaktion a) einzelsträngig gemacht werden. Eine evtl. notwendige Strangtrennung kann durch Behandlung mit Alkali, aber auch thermisch, enzymatisch oder mittels chaotroper Salze geschehen.

Die Verwendung von Reaktionen a), die von der Anwesenheit eines spezifischen Starters abhängig sind, ist wegen der erhöhten Spezifität besonders bevorzugt. Solche spezifischen Starter bewirken, daß das Enzym nur auf die Nukleinsäure wirkt, welche diesen Starter gebunden hat. Der Starter ist bevorzugt ein sogenannter spezifischer Primer P1, ein Promotor oder eine Replikationsinitiationsregion.

Spezifische Primer P1 sind besonders Oligonukleotide oder modifizierte Oligonukleotide, die eine im wesentlichen zu der Nukleinsäure A komplementäre Nukleotidsequenz aufweisen. Modifizierte Oligonukleotide sind beispielsweise Oligonukleotide, die am 3'-Ende ein Dideoxynukleotid enthalten. Solche Oligonukleotide können enzymatisch hergestellt werden. Als template-Nukleinsäure A kann direkt die target-Nukleinsäure eingesetzt werden.

Die Verwendung solcher Primer P1 setzt also voraus, daß zumindest ein Teil der Sequenz der Nukleinsäure bekannt ist. Die Sequenz des Primers wird ferner so gewählt, daß er an einem seiner Enden, bevorzugt am 3'-Ende, kürzer ist als die Nukleinsäure. Diese weist daher über das 3'-Ende des Primers hinaus ein überstehendes einzelsträngiges Teilstück auf.

In den Reaktionsschritt können pro nachzuweisendem Nukleinsäureeinzelstrang ein oder mehrere spezifische Primer eingesetzt werden. Im Fall mehrerer Primer überlappen sich die Bereiche auf der Nukleinsäure, mit denen die Primer hybridisieren können, bevorzugt nicht, und besonders bevorzugt liegen zwischen diesen Bereichen einzelsträngige Bereiche der Nukleinsäure A. Der Primer kann neben einem zu der template-Nukleinsäure A im wesentlichen komplementären Teil, bevorzugt am 5'-Ende, auch noch eine Nukleotidsequenz S1 beinhalten, die nicht mit der Nukleinsäure, insbesondere in dem an den komplementären Bereich anschließenden Bereich, hybridisieren kann.

Diese Sequenz S1 kann beispielsweise einzel- oder doppelsträngig sein und auch eine Erkennungssequenz für ein Enzym enthalten. Dies kann z.B. eine Restriktionsschnittstelle sein. Auch kann der Primer im Hinblick auf effektives Priming beispielsweise ein Protein gebunden enthalten, welches von einem Enzym E, vorzugsweise einer Polymerase, erkannt wird.

Damit die Reaktion a) ablaufen kann, müssen die Nukleinsäure A und der Primer P1 im komplementären Bereich zunächst jeweils von gegebenenfalls anwesenden Komplementärsträngen getrennt werden. Diese Trennung kann nach bekannten Methoden, beispielsweise thermisch oder nichtthermisch erfolgen. Die nichtthermische Denaturierung ist bevorzugt. Reaktion a) wird dann unter Bedingungen gestartet, bei denen A und P1 miteinander hybridisieren können.

Bei Verwendung eines Primers als Starter wird der Probe außerdem ein Enzym E zugegeben, welches in einer primerabhängigen Reaktion zu A komplementäre Nukleinsäure synthetisieren kann. Dazu gehören insbesondere Polymerasen. Ihre Substratspezifität richtet sich nach der Nukleinsäure A. Ist A RNS, so kommen insbesondere RNS-abhängige DNS-Polymerasen, wie reverse Transkriptase (z.B. aus AMV oder M-MuLV) in Frage. Ist A DNS, so sind DNS-abhängige DNS-Polymerasen, wie Klenow-Enzym der Taq-DNS-Polymerase bevorzugt.

Ebenfalls der Probe zugegeben werden Desoxyribonukleosidtriphosphate (dNTP), von denen mindestens eines markiert ist.

Produkt der Polymerasereaktion ist eine markierte Desoxyribonukleinsäure B, in welche der Primer P1 sowie das markierte Desoxyribonukleosidtriphosphat eingebaut sind. Diese Nukleinsäure B ist gleich groß oder kleiner als das template, weist jedoch einen Bereich auf, der zu mindestens einem Teil im wesentlichen komplementär zum template ist.

Diese Nukleinsäure B kann nun direkt in Schritt b) eingesetzt werden. Vorteilhaft wird sie jedoch nochmals als template-Nukleinsäure einer Reaktion analog zu Schritt a) unterworfen. Hierzu wird der Probe ein Primer P2 zugesetzt, der im wesentlichen komplementär zu einem Teil der Nukleinsäure B, bevorzugt zu einem Teil des neu aus dNTPs gebildeten Bereiches, ist. Das Primerpaar P1 und P2 erfüllen bevorzugt die in EP-A-0 201 184 geschilderten Bedingungen. Bevorzugt werden P1 und P2 gleichzeitig zu dem template A zugegeben.

Um eine noch höhere Empfindlichkeit zu erlangen, ist es möglich, Reaktion a) noch mehrere Male, bevorzugt 1-60, besonders bevorzugt 20-60 mal, durchzuführen, wobei jeweils die Produkte der Reaktion erneut in Reaktion a) eingesetzt werden. Dadurch ergibt sich theoretisch eine nahezu exponentielle Vermehrung an markierten Nukleinsäuren. Es werden dabei beide Nukleinsäurestränge gebildet, wobei die Länge durch die nicht verlängerbaren Enden der Primer P1 und P2 festgelegt ist. Es ist analog zu EP-A-0 201 184 möglich, in den späteren Durchläufen von Reaktion a) auch sogenannte nested Primer zu verwenden, deren Sequenz so gewählt ist, daS die enstehenden Nukleinsäuren kleiner sind als die zunächst gebildeten. Vor jedem Durchlauf von Reaktion a) ist es zweckmäßig, die in Reaktion a) gebildeten Doppelstränge aus A und B zu trennen. Dies kann thermisch oder nicht thermisch geschehen. Es müssen jeweils erneut Primer P1 und P2 anwesend sein.

Der Starter kann auch ein Promotor sein. Ein Promotor ist eine Nukleotidsequenz, die von einer RNS-Polymerase erkannt wird und diese veranlaßt, einen zu der auf den Promotor folgenden Nukleotidsequenz komplementären Nukleinsäurestrang B zu synthetisieren. Dabei wird neben den nicht markierten NTPs auch das markierte NTP in den gebildeten Nukleinsäurestrang eingebaut.

Geeignete Promotorn sind bekannt, beispielsweise RNS-Polymerase-Bindungsstellen aus Bakterienphagen wie T3, T7 oder SP6 (Melton et al.: NAR 12, 1984, 7035-56; Pfeiffer & Gilbert: Protein Sequences and DNA-Analysis I, 1988, 269-280; Uhlenbeck et al.: Nature 328, 1987, 596-600).

Der Promotor ist in einer bevorzugten Ausführungsform des Nachweisverfahrens Teil eines spezifischen Primers zur Herstellung der template-Nukleinsäure A aus der target-Nukleinsäure. Dieser Primer hat eine Nukleotidsequenz S1, die im wesentlichen komplementär zu einem Teil der target-Nukleinsäure ist, und eine Nukleotidsequenz S2, die von einer RNS-Polymerase erkannt wird und mindestens eine Promotor-sequenz beinhaltet. In einer ersten Reaktion wird mit dem Primer und einer von der Art der target-Nukleinsäure abhängigen DNS-Polymerase und dNTPs ein zu der target-Nukleinsäure mindestens teilweise komplementärer Nukleinsäurestrang A1 gebildet, der den Primer mit dem Promotor beinhaltet. Falls nicht bereits mit dem Primer verbunden, wird das neu gebildete Nukleinsäurestück durch Zugabe eines weiteren Enzyms, bevorzugt einer Ligase, z.B. E.coli-DNS-Ligase mit dem Primer kovalent verknüpft. Die Ligase kann auch thermisch stabil sein. A1 ist bevorzugt kürzer als die target-Nukleinsäure.

Bevorzugt wird in dieser Transkriptionsreaktion ein zweiter zum target-Nukleinsäurestrang komplementärer Primer P3 eingesetzt und die Lücke zwischen beiden Primern geschlossen, beispielsweise durch eine Gap-Filling-Reaktion. Der Einsatz markierter dNTPs ist hier noch nicht erforderlich, da sich diese Maßnahme im erfindungsgemäßen Verfahren in einer nur geringen Verstärkung des Meßsignals auswirkt, aber möglich.

Ist die target-Nukleinsäure RNS, so wird diese bevorzugt in einem folgenden Schritt selektiv abgebaut. Dazu sind bekannte Verfahren geeignet, beispielsweise die Behandlung mit Alkali oder mit RNAsen. Anschließend wird ein zu A1 komplementärer Nukleinsäurestrang A2 gebildet. Dazu wird der Reaktionsmischung ein Primer P2 zugesetzt, der zu einem Teil, bevorzugt einem neu gebildeten Teil des Stranges A1 komplementär ist. Bevorzugt enthält der Primer P2 ebenfalls die Promotorsequenz S2. Dieser wird, wie oben bei A1 beschrieben zu A2 verlängert. Solche Verfahrensschritte sind z.B. in der EP-A-0 329 822, DE-A-37 26 934, WO 88/10315, WO 87/06270, EP-A-0 310 229 und EP-A-0 373 960 beschrieben, weshalb hier auf diese Offenbarungen vollinhaltlich Bezug genommen wird. Insbesondere in Bezug auf Details, die zur reversen Transkription von RNS oder Transkription von DNS hilfreich und erforderlich sind, wird auf diese Offenbarung verwiesen.

Besonders bevorzugt enthält die Probe bei dieser Ausführungsform außerdem den zu der target-Nukleinsäure komplementären Strang, wobei P1 und P2 gleichzeitig verlängert werden.

Bei der genannten Ausführungsform wird der so vorbehandelten Probe anschließend erfindungsgemäß eine Promotor-spezifisch gesteuerte DNS-abhängige RNS-Polymerase eingesetzt. Solche Polymerasen sind z.B. T3, T7 oder SP6 RNS-Polymerase. Mit ihrer Hilfe werden aus NTPs und dem markierten NTP markierte Nukleinsäuren B gebildet. Die doppelsträngige Nukleinsäure A1/A2 dient dabei als template-Nukleinsäure, bevorzugt mehrmals.

Bevorzugte NTPs sind Ribonukleosidtriphosphate. Da bei dieser Variante der Reaktion a) einzelsträngige Nukleinsäuren B gebildet werden, ist eine Denaturierung zur Strangtrennung nicht unbedingt erforderlich, aber möglich.

Eine bevorzugte Ausführungsform ist das Vorgehen nach DE-A-4010465, jedoch unter Verwendung nachweisbar markierter Ribonukleosidtriphosphate.

Der Starter ist bevorzugt eine Replikationsinitiationsregion (RIR). Eine Replikationsinitiationsregion ist beispielsweise eine Nukleotidsequenz, die von einem Replikationsenzym bzw. - enzymkomplex, beispielsweise einer DNS-abhängigen DNS-Polymerase, wie von φ 29 (P2, P3) erkannt wird. Besonders bevorzugt ist der Fall, daß die RIR ein Teil eines spezifischen Primers ist. Dieser Primer hat eine Nukleotidsequenz S1, die im wesentlichen komplementär ist zu einem Teil der target-Nukleinsäure und daran anschließend eine Sequenz S2, welche eine Erkennungsstelle, bevorzugt Proteine, für einen Replikationskomplex, aufweist. Ein solcher Primer wird im folgenden Adaptor Ad genannt. Die Reaktion wird eingeleitet, indem die target-Nukleinsäure, welche die eigentlich nachzuweisende Spezies ist, mit Ad unter Hybridisierungsbedingungen umgesetzt wird. Besonders bevorzugt ist der Einsatz von zwei Adaptoren Ad1 und Ad2 pro target-Nukleinsäureeinzelstrang, wobei die targetspezifischen Sequenzen mit voneinander räumlich getrennten Sequenzen der einzelsträngigen target-Nukleinsäure hybridisieren können und die targetspezifischen Sequenzen des Adaptoren auf der target-Nukleinsäure aufeinander zu gerichtet sind.

Die einzelsträngige Lücke zwischen den Adaptoren wird durch eine gap-filling Reaktion (Maniatis et al., Molecular Cloning, 1982, CSH) aufgefüllt. Der Einsatz von markierten NTPs ist nicht erforderlich, aber möglich. Dadurch entsteht ein Nukleinsäuredoppelstrang, welcher einen Strang der target-Nukleinsäure und einen Strang der neugebildeten Nukleinsäure A enthält. Dieser enthält mindestens eine, bevorzugt 2 Replikationsinitiationsregionen, welche besonders bevorzugt in entgegengesetzter Replikationsrichtung angeordnet sind.

Aus diesem Doppelstrang kann nun auf erfindungsgemäße Weise durch Replikation ohne weiteren Zusatz von Adaptoren unter Verwendung von markierten und unmarkierten Nukleosidtriphosphaten in Reaktion a) eine markierte Nukleinsäure B hergestellt werden. Diese enthält wiederum mindestens eine, bevorzugt 2, Replikationsinitiationsregionen und kann daher erneut in Reaktion a) als template-Nukleinsäure eingesetzt werden. Somit wird eine erhebliche Vemehrung von B erreicht. Für den Fall, daß beide Adaptoren je eine RIR aufweisen, oder zwei zueinander komplementäre Nukleinsäuren A in Reaktion a) eingesetzt werden, können zwei zueinander komplementäre Nukleinsäuren B entstehen. Diese müssen vor Schritt b) voneinander durch Denaturierung getrennt werden.

Nach Schritt a) wird in dem erfindungsgemäßen Verfahren die Reaktionsmischung einer nichtthermischen Denaturierung (Reaktion e)) unterzogen. Auch falls Schritt a) mehrfach durchgeführt wird, findet die nichtthermische Denaturierung direkt vor Schritt b) statt. Insbesondere werden dabei Nukleinsäuredoppelstränge voneinander getrennt. Nichtthermische Denaturierung bedeutet, daß die Temperatur zur Denaturierung nicht über 50°C, bevorzugt nicht über 37°C, bevorzugt in einem Bereich zwischen 22 und 37°C stattfindet. Bei der Denaturierung werden eventuell vorliegende Nukleinsäuredoppelstränge voneinander getrennt, sodaß Nukleinsäure B als Einzelstrang vorliegt. Dies kann beispielsweise durch Veränderung der Stringenz geschehen aber auch durch Enzyme, beispielsweise Helicase, recA-Protein, E.coli ssb-Protein, Gen-32-Protein, und durch Alkali oder durch Zusatz von doppelstrangstabilisierenden Chemikalien, wie z.B. organischen Substanzen, wie Harnstoff, Formamid (Konzentration > 70%, NAR 1977, Vol. 4, 5, 1539-1553) oder chaotropen Salzen. Chaotrope Salze sind in J. Biol. Chem. 1966, 241/17, 4030-4042 J. Amer. Chem. Soc. USA 1962, 84/8, 1329-1338 oder Anal. Biochem. 129, 357-364 (1983) beschrieben. Besonders bevorzugt im Sinne der Erfindung sind NaJ, Guanidinumthiocyanat oder NaClO₄ . Die zur Denaturierung erforderliche Konzentration dieser Salze kann durch einfache Versuche, ermittelt werden. Sie beträgt jedoch z.B. für NaJ bevorzugt ca. 12.2 mol/l, für Guanidiniumsalze ca. 4 mol/l und für NaClO₄ ca. 7 mol/l.

Als besonders vorteilhaft hat sich hier die alkalische Denaturierung erwiesen. Dazu wird die Reaktionsmischung aus Reaktion a) auf einen pH im Bereich von 10 bis 14, bevorzugt von 12 bis 14, gebracht. Dies kann beispielsweise durch Zugabe einer Lösung von NaOH oder KOH in Wasser geschehen.

Die Temperatur der Mischung liegt während der Inkubation von 1 bis 30 min bevorzugt von 5 bis 10 min, in einem Bereich von 17 bis 50°C, bevorzugt von 22 bis 37°C.

Im folgenden Reaktionsschritt b) wird Nukleinsäure B mit der Sonde C so zur Reaktion gebracht, daS sie zusammen ein Nukleinsäurehybrid D bilden.

Als Nukleinsäuresonde C kommen insbesondere Oligo- bzw. Poly-Nukleotide mit einer Länge von 6 bis 5000, bevorzugt 15 bis 2000 in Frage. Die Sonde C kann ein Plasmid, ein Nukleinsäurefragment oder ein Oligonukleotid sein. Es kann sich um RNS oder DNS handeln. Nukleinsäuresonde C wird im Überschuß zu der erwarteten Menge an Nukleinsäure B zu der Reaktionsmischung vorteilhaft in Form einer wässrigen Lösung zugegeben. Die Sonde C weist eine Nukleotidsequenz auf, die im wesentlichen komplementär zu B ist, und die für B spezifisch ist, also nicht oder nur in sehr geringem Umfang mit in der Probe vorhandenen oder neu gebildeten Nukleinsäuren hybridisiert, die nicht nachgewiesen werden sollen. Durch die Kombination der Verwendung spezifischer Primer und der Hybridisierung mit einer spezifischen Sonde wird das erfindungsgemäße Verfahren besonders selektiv.

C kann eine doppelsträngige Nukleinsäure sein, deren einer Strang C1 komplementär zu einem Teil von B ist. Bevorzugt ist der andere Strang C2 der Nukleinsäuresonde zu anderen Nukleinsäuren B komplementär, insbesondere zu solchen, die bei Durchführung von Reaktion a) mit B als template-Nukleinsäure entstehen. Die Denaturierung von C kann dabei separat von B vorgenommen werden. Es ist jedoch bevorzugt, C zusammen mit B zu denaturieren. Dies gilt insbesondere für die alkalische Denaturierung. Dazu wird C bevorzugt vor der Alkalisierung der Reaktionsmischung aus a) zugegeben. Nach einer Inkubationszeit wird die Mischung auf einen pH von 5.0 bis 8.5, bevorzugt 7-8, gebracht, worauf sich die Nukleinsäurehybride D aus B und C1 bzw. C2 bilden.

Bevorzugt ist der Fall, daß es sich bei C um eine einzelsträngige Nukleinsäuresonde C1 handelt und der zu C1 komplementäre Strang C2 nicht zugesetzt wird. Zwar ist es dann auch möglich, C1 vor Denaturierung von B zuzugeben. Besonders bevorzugt ist dann jedoch im Fall der alkalischen Denaturierung von B, zuerst nur B alkalisch zu inkubieren und danach eine saure Lösung der Sonde C1 zuzusetzen. Der pH der sauren Lösung von C1 liegt in einem Bereich von 3.0 bis 6.5, bevorzugt 4.5 bis 6.0, ohne daß eine merkliche Instabilität der Sonde festzustellen ist. Die schwach saure Lösung sollte nach Zugabe zur Hybridisierungslösung zur Einstellung eines pH von 5.0 bis 8.0 ausreichen. Die Lösung kann auch in diesem Bereich gepuffert sein und weitere zur Hybridisierung hilfreiche Reagenzien enthalten, wie z.B. SSC, Formamid oder Blockierungsreagenzien für nicht nachzuweisende Nukleinsäuren. Der pH der Mischung nach Zugabe von C1 sollte auch hier im Bereich von 5.0 bis 8.5 liegen. Die Feinregulierung des pHs kann beispielsweise durch die Menge an Hybridisierungslösung sowie deren pH vorgenommen werden.

Säuren, die zur Neutralisation bzw. als Bestandteil der Hybridisierungslösung verwendet werden können, sind beispielsweise Salzsäure oder Ameisensäure; bevorzugt sind jedoch Essigsäure oder Phosphorsäure. Der pH der Hybridisierungslösung vor Zugabe zur Probe liegt zwischen 3.0 und 6.5. Die Konzentration am Beispiel der Essigsäure liegt bevorzugt zwischen 0.05 und 0.5 mol/l, besonders bevorzugt 0.1 und 0.2 mol/l.

Einzelsträngige Nukleinsäuresonden C1 können beispielsweise durch chemische Nukleinsäuresynthese gemäß DE-A- 39 16 871 oder auch gemäß EP-B-0 184 056 hergestellt werden.

Diese Ausführungsform hat den Vorteil, daß durch Verwendung der Sondenlösung zur Neutralisation der Probenlösung ein Pipettierschritt eingespart werden kann. Außerdem hat es sich erwiesen, daß die Zugabe einer konzentrierten Säure zu der alkalibehandelten Probe mit hohem Proteingehalt zu Verklumpungen führt, weshalb die vorgeschlagene Vorgehensweise besonders vorteilhaft ist.

Falls vor Schritt b) eine Denaturierung mittels doppelstrangdestabilisierenden Substanzen vorgenommen wurde, können Bedingungen für die Hybridisierung mit der Sonde C eingestellt werden, indem das Gemisch verdünnt wird.

Sonde C enthält pro Nukleinsäurestrang eine oder mehr zur Immobilisierung befähigende (immobilisierbare) Gruppen I.

Zur Immobilisierung befähigende Gruppen I sind beispielsweise chemische Gruppen, die kovalent, beispielsweise über eine chemische oder eine Photoreaktion an eine feste Phase gebunden werden können, oder Gruppen bzw. Molekülteile, die über gruppenspezifische Wechselwirkungen von einem anderen Molekül oder Molekülteil erkannt und gebunden werden können. Solche Gruppen sind daher z. B. Haptene, Antigene und Antikörper, Nukleotidsequenzen, Rezeptoren, Regulationssequenzen, Glykoproteine, beispielsweise Lectine, oder auch die Bindungspartner von Bindeproteinen, wie Biotin oder Iminobiotin. Bevorzugt sind Vitamine und Haptene, besonders bevorzugt sind Biotin, Fluorescein oder Steroide, wie Digoxigenin oder Digoxin. Für die Erfindung ist es wichtig, daß in jedem Hybrid D die immobilisierbare Gruppe der Sonde sich von der nachweisbaren Gruppe der Nukleinsäure B unterscheidet.

Die Mischung wird anschließend mit einer festen Phase F in Kontakt gebracht, welche das Hybrid D über die immobilisierbaren Gruppen der Nukleinsäuresonde C spezifisch binden kann.

Die Art der Festphase richtet sich nach der zur Immobilisierung befähigenden Gruppe I. Bevorzugt weist sie eine immobilisierende Gruppe R auf, die eine bindende Wechselwirkung mit I eingehen kann. Ist die immobilisierbare Gruppe beispielsweise ein Hapten, dann kann eine Festphase verwendet werden, die an ihrer Oberfläche Antikörper gegen dieses Hapten aufweist. Ist die immobilisierbare Gruppe ein Vitamin, wie z. B. Biotin, dann kann die Festphase bindende Proteine, wie Avidin oder Streptavidin immobilisiert enthalten. Besonders bevorzugte Reste I und R sind Biotin und Streptavidin. Die Immobilisierung über eine Gruppe an der modifizierten Nukleinsäure ist besonders vorteilhaft, da sie unter milderen Bedingungen stattfinden kann als beispielsweise Hybridisierungsreaktionen.

Bevorzugt wird zur Immobilisierung der gebildeten Nukleinsäuren die Reaktionsmischung in ein Gefäß gefüllt, welches an seiner Oberfläche mit der immobilisierbaren Gruppe reagieren kann. Die Hybridisierungsreaktion mit der Sonde findet gleichzeitig mit der Immobilisierung statt. Das Gefäß kann beispielsweise eine Küvette, ein Röhrchen oder eine Mikrotiterplatte sein. Es ist jedoch auch möglich, eine Festphase in Form eines porösen Materials, wie einer Membran, eines Gewebes oder eines Vlieses, zu verwenden, auf welche die Reaktionsmischung aufgegeben wird. Ebenso ist die Verwendung von Perlen, sogenannten beads, oder Latex-Partikeln möglich. Die feste Phase sollte mindestens soviele Bindungsstellen für die immobilisierbare Gruppe der Sonde haben, wie Nukleinsäurehybride D und damit Nukleinsäuren B vorhanden sind.

Die Herstellung einer bevorzugten festen Phase ist in der EP-A-0 344 578 beschrieben, auf welche vollinhaltlich Bezug genommen wird.

Nach einer Inkubationszeit, während der die Immobilisierungsreaktion stattfindet, wird die flüssige Phase aus dem Gefäß, dem porösen Material oder den pelletierten beads entfernt. Die Festphase kann anschließend mit einem geeigneten Puffer gewaschen werden, da die Bindung der Hybride D an der Festphase sehr effizient ist. Das erfindungsgemäße Verfahren erlaubt dabei die Verwendung besonders weniger Waschschritte, da die schwer abtrennbaren Sonden im Gegensatz zu im Stand der Technik verwendeten detektierbaren Sonden nicht unbedingt vollständig entfernt werden müssen, oder führt zu vergleichsweise geringen Hintergrundsignalen.

Die Menge der an die Festphase gebundenen modifizierten Nukleinsäuren kann auf im Prinzip bekannte Weise bestimmt werden, wobei sich die durchzuführenden Schritte nach der Art der nachweisbaren Gruppe richten. Bei direkt nachweisbaren Gruppen, beispielsweise Fluoreszenzlabeln, wird die Menge an Label fluorometrisch bestimmt. Ist die nachweisbare Gruppe ein Hapten, so wird die modifizierte Nukleinsäure bevorzugt mit einem markierten Antikörper gegen das Hapten umgesetzt, wie analog in der EP-A-0324474 beschrieben. Die Markierung kann beispielsweise eine Enzymmarkierung, wie β-Galaktosidase, alkalische Phosphatase oder Peroxidase sein. Im Fall der Enzymmarkierung wird die Menge an Nukleinsäure über die meist photometrische, chemoluminometrische oder fluorometrische Verfolgung einer Reaktion des Enzyms mit einem chromogenen, chemoluminogenen oder fluorogenen Substrat gemessen. Es ist jedoch auch elektrochemische Verfolgung der Reaktion möglich, wenn ein Redoxenzym als Markierung verwendet wird, oder die Verfolgung einer pH-Wertänderung mittels einer pH-Elektrode. Das Meßsignal ist ein Maß für die Menge an ursprünglich vorhandener target-Nukleinsäure.

Der Nachweis der Nukleinsäure kann sowohl qualitativ als auch quantitativ geführt werden. Im Falle einer quantitativen Auswertung hat es sich als zweckmäßig herausgestellt, mindestens einen Vergleichsversuch mit einer Probe bekannten Nukleinsäuregehalts durchzuführen. Die Erstellung einer Eichkurve ist möglich und empfehlenswert.

In einer Ausführungsform des Verfahrens unter Verwendung der PCR werden der Probe als Primer P1 und P2 Oligonukleotide zugegeben. P1 ist hierbei komplementär zu einem Teil des Nukleinsäureeinzelstrangs A, welcher die target- und gleichzeitig die template-Nukleinsäure darstellt. P2 ist homolog zu einem davon entfernten Teil von A. Das Gemisch wird nun, wie in EP-A-0 201 184 beschrieben, behandelt, wobei jedoch neben den unmodifizierten Desoxymononukleotidtriphosphaten auch ein digoxigeninmarkiertes oder fluoresceinmarkiertes Desoxymononukleotidtriphosphat eingesetzt wird. Es werden bevorzugt 20-30 Amplifikationszyklen durchgeführt. Danach werden doppelsträngige biotinmarkierte Sonde C und Natronlauge zu einem pH von 10-14 zugegeben. Die Mischung wird bei ca 37°C inkubiert und anschließend mit einer Lösung von Hybridisierungshilfsstoffen bei ca. pH 5 versetzt, sodaß sich ein pH von ca. 7-8.5 ergibt. Die Mischung wird in ein Streptavidin-beschichtetes Gefäß umgefüllt und erneut inkubiert. Die Lösung wird entfernt und das Gefäß gewaschen. Es wird ein Konjugat aus Antikörper gegen Digoxigenin und einem Enzym zugegeben und erneut inkubiert. Nach Entfernen der Lösung und Waschen des Gefäßes wird mit einem chromogenen Substrat des Enzyms umgesetzt und die Farbbildung beobachtet.

Eine bevorzugte Ausführungsform ist eine Variante des in DE-A-39 29 030 beschriebenen Replikationsverfahrens. Auf die Offenbarung der DE-A-39 29 030 wird hiermit vollinhaltlich Bezug genommen. Zur Durchführung des Verfahrens wird die Probe, welche die einzelsträngige target-Nukleinsäure enthält, mit zwei Adaptoren, die zu dem gleichen Strang komplementär sind und deren mit A hybridisierenden Enden aufeinander zu gerichtet sind und deren nicht hybridisierenden Enden eine Replikationsinitationssequenz für Ø 29-Polymerase enthalten, versetzt. Unter Hybridisierungsbedingungen wird die zwischen den Adaptoren befindliche Lücke durch gap-filling mittels DNA-Polymerase oder reverse Transskriptase (als Enzym E1) und Ligase-Reaktion geschlossen. Die hierbei verwendeten Desoxy-mononukleosidtriphosphate können unmodifiziert sein, aber bevorzugt jedoch ist eines von ihnen ein digoxigeninmarkiertes Monodesoxyribonukleosidtriphosphat. Zur Durchführung der Replikation wird das Replikationssystem von Ø 29 (P2, P3 und ATP) sowie neben unmodifizierten Mononukleosidtriphosphaten, falls nicht schon bei der gap-filling-Reaktion verwendet, ein digoxigenin- oder fluorescein-markiertes Monodesoxyribonukleo-sidtriphosphat zugegeben. Die durch Replikation gebildeten nachweisbar markierten Nukleinsäuren B dienen ohne weitere Zugabe von Adaptoren erneut als template-Nukleinsäuren zur Bildung weiterer Nukleinsäuren B. Daher kommt es mit der Zeit theoretisch zu einer exponentiellen Vermehrung der nachweisbar markierten Nukleinsäuren B. Sobald genügend Nukleinsäuren entstanden sind, wird die Mischung einem nicht-thermischen Denaturierungsschritt unterzogen und mit einer bevorzugt einzelsträngigen biotinmarkierten Sonde C1 in einer Lösung mit Hybridisierungsreagenzien bei pH 3.0-6.5 bis zu einem pH von 5.0-8.5 bevorzugt 7.0-8.0 versetzt. Durch Einführung des nichtthermischen Denaturierungsschritts wird es möglich, das gesamte Nachweisverfahren in einem Temperaturbereich zwischen 17 und 50°C und bevorzugt bei einer einzigen Temperatur durchzuführen. Die Mischung wird in ein streptavidin-beschichtetes Gefäß umgefüllt und inkubiert. Nach Absaugen der Flüssigkeit und Waschen des Gefäßes wird eine Lösung eines Konjugats aus einem enzymmarkierten Antikörper gegen Digoxigenin bzw. Fluorescein zugegeben und wieder inkubiert. Nach Absaugen der Lösung und Waschen wird mit einer Lösung eines chromogenen Substrats versetzt und die Farbbildung beobachtet.

In den Verfahren des Standes der Technik wurden bisher immer nachweisbar markierte Sonden eingesetzt. Eine vollständige Abtrennung der nicht hybridisierenden Sonden war für die Genauigkeit des Meßergebnisses erforderlich, jedoch relativ aufwendig.

Das erfindungsgemäße Verfahren umgeht diesen Nachteil, indem anstelle der Hybridisierung von markierten Sonden und deren Bestimmung die Anwesenheit eingebauter markierter Mononukleotide gemessen und als Maß für die Anwesenheit bzw. die Menge an nachzuweisenden Nukleinsäuren genommen wird. Die Abtrennung nicht eingebauter markierter Mononukleotide ist nach dem vorliegenden Verfahren besonders einfach und vollständig zu erreichen, da sie weder an die Nukleinsäuren noch an die Oberfläche gebunden werden. Ein Überschuß an immobilisierbarer Sonde C hingegen stört die Bestimmung nicht, da die immobilisierbaren Gruppen von Sonde C nicht als Markierung verwendet werden und somit nicht zum Meßergebnis beitragen. Insbesondere ist die Bindekapazität der festen Phase besser kalkulierbar als beim Einbau von immobilisierbaren NTPs.

Das erfindungsgemäße Verfahren ist daher sehr sensitiv und selektiv, außerdem kann es in kurzer Zeit durchgeführt werden.

Das erfindungsgemäße Verfahren eignet sich zum Nachweis von Nukleinsäuren als solchen, von nukleinsäurehaltigen Organismen z.B. Bakterien, Viren und Zellen und zum Nachweis von Nukleinsäureveränderungen, wie Mutationen oder Chromosomentranslokationen und zur Lokalisierung und zum Expressionsgrad von Genen. Sowohl Ribonukleinsäuren als auch Desoxyribonukleinsäuren können nachgewiesen werden. Auch die Identifizierung von punktuellen Unterschieden in Nukleinsäuren ist möglich, wenn die Ergebnisse von Experimenten verglichen werden, in denen in Reaktion a) einmal ein Primer eingesetzt wird, der an einem 3'-Ende zu der Nukleinsäure A komplementär ist, zum anderen ein Primer eingesetzt wird, der an seinem 3'-Ende gerade nicht zu Nukleinsäure A komplementär ist, aber dennoch mit ihr hybridisiert. Eine Verlängerung des Primers und damit Bildung der Nukleinsäure B wird nur im ersten Fall möglich sein. Die Auswahl solcher Primer ist beispielsweise in PNAS 86, 2757 (1980) beschrieben.

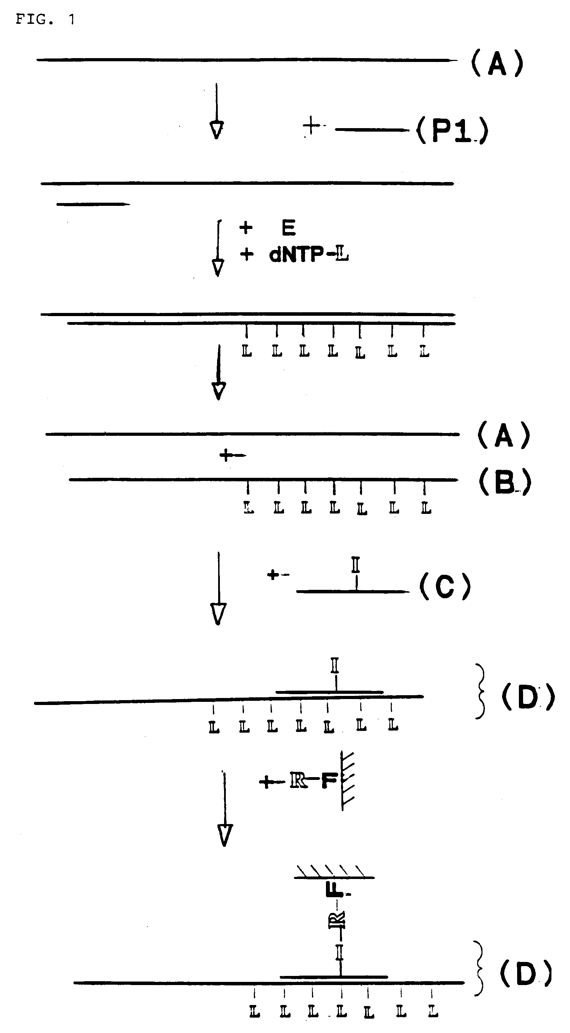

Figur 1 zeigt schematisch den Ablauf einer Ausführungsform unter Verwendung einer Primerelongation (Verwendung nur eines Primers P1).

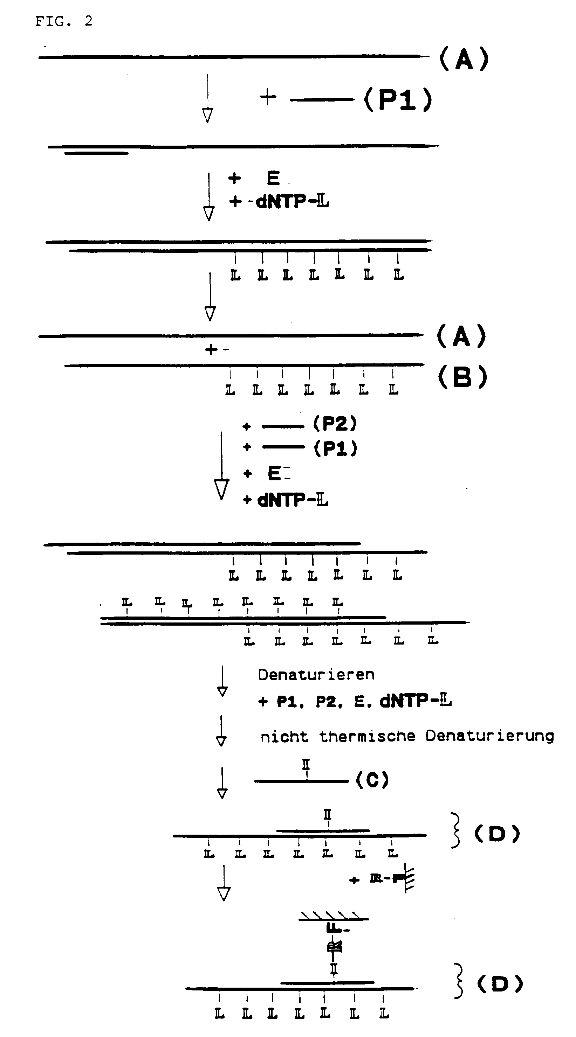

Figur 2 zeigt schematisch den Ablauf einer Ausführungsform unter Verwendung des PCR-Prinzips mit zwei Primern, wobei die zunächst gebildeten Nukleinsäuren B erneut als template-Nukleinsäure eingesetzt werden.

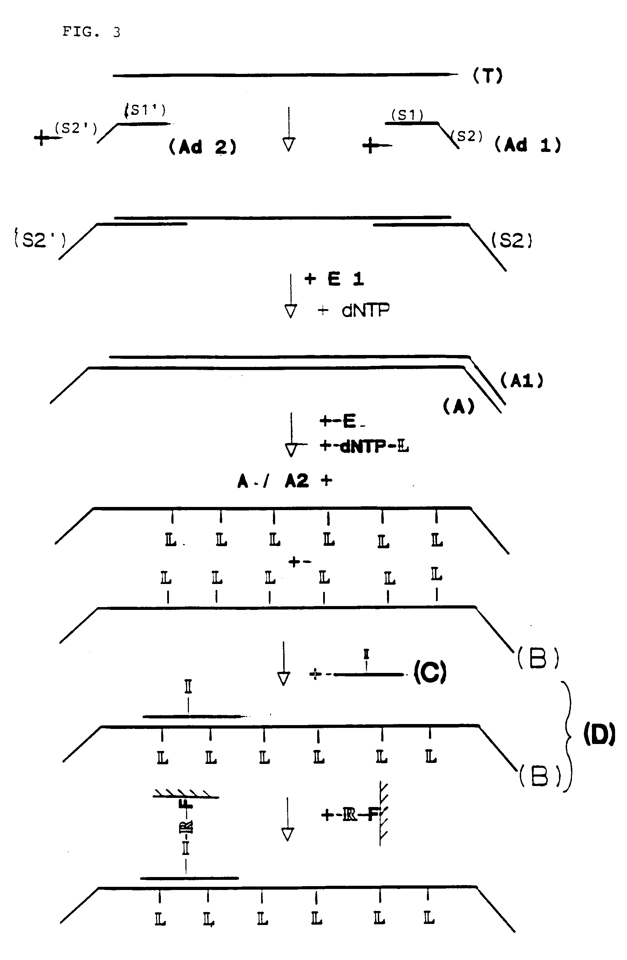

Figur 3 zeigt schematisch den Ablauf der bevorzugten Ausführungsform unter Verwendung einer Replikationsamplifikation.

Figur 4 zeigt in Kurve I das Ergebnis eines Experiments nach Figur 1. Aufgetragen sind gegeneinander die in der Lösung gemessene Extinktion gegen die Menge an nachzuweisender Nukleinsäure A; Kurve II gibt das Ergebnis ohne Anwesenheit der nachzuweisenden Nukleinsäure wieder.

Fig. 5 zeigt eine Eichkurve 1 für eine Bestimmung von HBV-DNA mittels PCR und einem einzelsträngigen biotinmarkierten Oligonukleotid gemäß Beispiel 2. Kurve 2 zeigt den Leerwert (ohne Zugabe von Bio-Oli 25).

Fig. 6 zeigt eine Eichkurve 3 für die Bestimmung von HBV in einem Verfahren gemäß Beispiel 3. Kurve 4 zeigt den Leerwert (ohne Zugabe von Bio-Oli 25).

### Bezugszeichen:

- A: template-Nukleinsäure
- A1: Gegenstrang von A
- P1: zu A komplementärer Primer
- E: Enzym/Enzymkomplex
- E1: DNA-Polymerase oder reverse Transskriptase
- L: nachweisbare Gruppe (Markierung)
- B: Produkt der Reaktion a)
- C: Nukleinsäuresonde
- I: immobilisierbare Gruppe
- D: Nukleinsäurehybrid aus B und C
- R: immobilisierende Gruppe
- F: feste Phase
- S1: zu A komplementäre Sequenz
- S1': andere zu A komplementäre Sequenz
- S2: Promotor- oder erste Replikationsinitiationssequenz (Replikationsursprung)
- S2': Promotor- oder zweite Replikationsinitiationssequenz (Replikationsursprung)
- Ad1: Adaptor 1, komplementär zur target-Nukleinsäure
- Ad2: Adaptor 2, komplementär zur target-Nukleinsäure

Die Erfindung wird durch die folgenden Beispiele näher erläutert:

### Beispiel 1

### Amplifikation der target-DNA unter Verwendung eines nachweisbar markierten Desoxyribonukleosidtriphosphats (Schritt a) )

Polymerase chain reaction wird mit klonierten HBV-spezifischen Sequenzen durchgeführt. Die verwendeten Primer binden an die Position 1937-1960 und 2434-2460 im HBV-Genom, d.h. eine Sequenz von 523 Nukleotiden wird amplifiziert (R. Sumazaki et al., 1989, J. Med. Virol, 27, 304-308). Die Konzentration der zu amplifizierten DNA im Plasmid entspricht in der hier eingesetzten Verdünnungsreihe 100 ag bis 100 pg. 30 PCR-Zyklen (2' 92°C; 2' 50°C; 2' 70°C) werden in 100 »l mit Primern, je 200 nM; KCl, 50 mM; Tris HCl pH 8,5, 10 mM; MgCl₂, 1,5 mM; Gelatine 100 »g/ml; dATP, 200 »m; dCTP, 200 »m, dGTP, 200 »m; dTTP, 150 »M; Digoxigenin-11-2'-desoxy-uridin-5'-dUTP (DIG-[11]-dUTP, Boehringer Mannheim), 50 »m; 2.5 U Thermus aquaticus (Taq) DNA-Polymerase durchgeführt.

### Nichtthermische Denaturierung (Schritt e))

20 »l dieses Gemisches nach PCR und 40 ng Biotin-markierte Proben-DNA (random-geprimte, klonierte HBV-DNA zwischen Position 27 und 2604, doppelsträngig) werden in einem Gesamtvolumen von 44 »l in 0,5 M NaOH 10'bei 37°C denaturiert.

### Hybridisierung mit Sonde C (Schritt b)) und Festphasenbindung (Schritt f)).

Anschließend wird durch Zugabe von 156 »l Hybridisierungslösung von pH 5 neutralisiert und in eine Streptavidin-beschichtete Mikrotiterplatte umpipettiert (Hybridisierungslösung = 62,5 mM Natriumphosphat, 6,25 x SSC [1 x SSC = NaCl, 0,15 M; Natriumzitrat, 0,015 M] und Formamid 62,5 %). Die Hybridisierung-/Wandbindungsreaktion wird 18 Stunden bei 37°C unter leichtem Schütteln durchgeführt.

### Detektion des Hybrids D (Schritte c), g) und h)).

Nach Entfernen der Flüssigkeit und 5x Waschen mit 0,025 % NaCl und 1 oo/o Kupfersulfat werden 200 mU/ml Anti-Digoxigenin-Meerrettich-Peroxidase-Konjugat zugegeben und 60'bei 37°C in Tris HCl, pH 7,5/10 mM; NaCL 0,9 %; BSA, 1 %; Pluronic T68 0,5 % inkubiert. Nach 5x waschen (gleiche Bedingungen wie oben) wird mit 0,1 % 2,2-Azino-di-[3-ethylbenzthiazol]-(ABTS) 60' bei 37°C inkubiert und die Extinktion bei 405 nm gemessen.

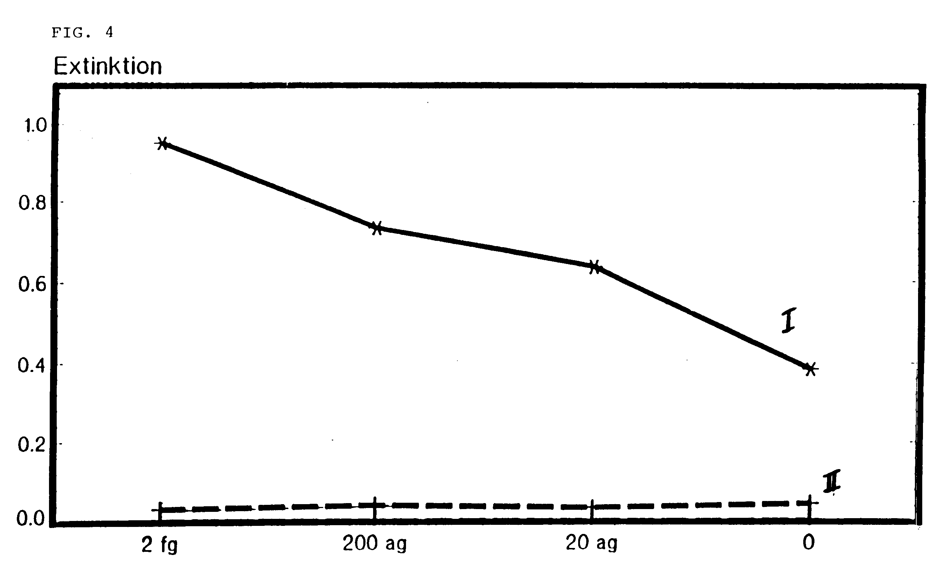

In Figur 4 sind die Extinktionen für diese Reaktion und für die Kontrollreaktion mit biomarkierter Probe gezeigt.

### Beispiel 2

Die Amplifikationsreaktion (Polymerase chain reaction) wird mit Kontrollserum (HBV-DNA negativ) durchgeführt, das mit HBV-spezifischer DNA angereichert ist. Aufgrund der Lage der gewählten PCR-Primer wird bei der Amplifikation ein 523 bp großes DNA-Fragment gebildet (PCR-Primer 1: Position 1937-1960, PCR-Primer 2: Position 2434-2460 im HBV-Genom, Literatur siehe Beispiel 1). Es wird eine Verdünnungsreihe des Plasmids in einem Bereich von 10 pg/ml bis 1,25 pg/ml in Kontrollserum hergestellt. Dies entspricht, bezogen auf den zu amplifizierenden DNA-Bereich des Plasmids, Mengen im Bereich von 40 ag bis 320 ag DNA, die in die Amplifikationsreaktion eingesetzt werden. Zur Alkalilyse werden 10 »l des mit Plasmid-DNA aufgestockten Kontrollserums mit 50 »l 10 mM Tris-HCl (pH 8,3) und 20 »l 0,5 N NaOH versetzt und 1 h bei 37°C inkubiert. Nach Neutralisation mit 20 »l 0,5 N HCl werden
10 »l des Lyseansatzes in die Amplifikationsreaktion eingesetzt. Die Reaktion erfolgt in einem Gesamtvolumen von 100 »l mit je 200 nM der PCR-Primer, je 200 »M dATP, dCTP und dGTP, 175 »M dTTP, 25 »M Digoxigenin-11-2'-dUTP (Boehringer Mannheim), 2,5 U Thermus aquaticus DNA-Polymerase, 50 mM KCl, 10 mM Tris-HCl (pH 8,3), 1,5 mM MgCl₂, 0,01 % Gelatine. Nach Überschichten der Lösung mit 100 »l Mineralöl erfolgt die Inkubation in einem Thermocycler (Perkin Elmer): 30 sec 92°C, 30 sec 50°C, 60 sec 70°C, insgesamt 30 Zyklen. Nach Beendigung der Amplifikation werden 40 »l des Ansatzes mit 10 »l 0,5 N NaOH versetzt und 10 min bei Raumtemperatur inkubiert. Die Neutralisation erfolgt durch Zugabe von 450 »l Hybridisierungslösung (50 mM Na-Phosphatpuffer, 0,75 M NaCl, 0,075 M Na-Citrat, 0,05 N HCl, 0,05 % RSA, pH 5,4) dem 220 ng/ml Biotin-markiertes Oligonukleotid (Bio-Oli 25, Biotin-markiert, gemäß Applied Biosystems, User Bulletin, DNA-Synthesizer Nr. 49, 1988, Position 2327-2356 im HBV-Genom), das komplementär zu einer Region des amplifizierten DNA-Bereiches ist. 200 »l des Hybridisierungsansatzes werden in einer Kavität einer mit Streptavidin beschichteten Mikrotiterplatte 3 h bei 37°C inkubiert. Nach Absaugen der Lösung wird anschließend 2 x 10 min bei 37°C mit 0,3 M NaCl, 0,03 M Na-Citrat, 0,2 % SDS und 1 x kurz bei Raumtemperatur mit 0,9 % NaCl gewaschen. 200 mU/ml anti-Digoxigenin-Antikörper-Meerrettichperoxidase-Konjugat in 100 mM Tris-HCl (pH 7,5), 0,9 % NaCl, 1 % RSA werden zugegeben und 30 min bei 37°C inkubiert. Nach 3maligem Waschen mit 0,9 % NaCl wird die Substratlösung (1,9 mM ABTS^{R}) zugefügt und die Extinktion bei 405 nm gemessen.

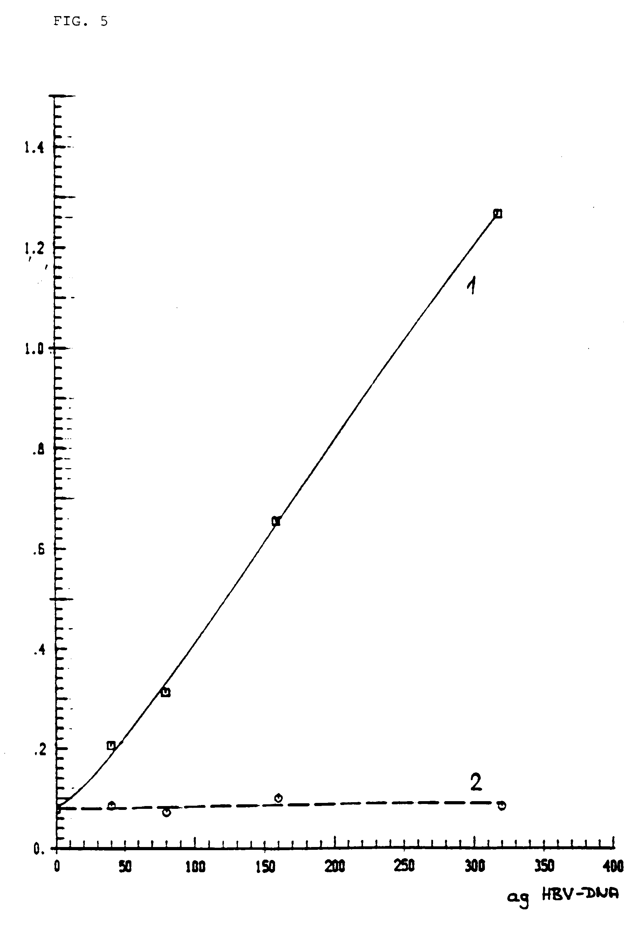

In Figur 5 sind die Ergebnisse der Reaktion graphisch dargestellt.

### Beispiel 3

HBe-positives Human-Plasma mit einem Virustiter von 1 x 10¹⁰ Hepatitis B-Viren pro mol wird in Normalserum verdünnt, so daß der Virusgehalt der Verdünnungen zwischen 0,25 x 10⁵/ml bis 2 x 10⁵/ml liegt. Zur Lyse der Viren werden 10 »l der Serumverdünnung mit 10 »l 0,2 N NaOH versetzt und 1 h bei 37°C inkubiert. Die anschließende Neutralisation erfolgt durch Zugabe von 30 »l Neutralisationslösung (100 mM KCl, 20 mM Tris-HCl (pH 8,3) ), 3 mM MgCl₂, 0,02 % Gelatine, versetzt mit 10 »l des Lyseansatzes verwendet. Die Reaktion erfolgt in einem Gesamtvolumen von 100 »l mit je 200 nM der beiden PCR-Primer, je 200 »m dATP, dCTP und dGTP, 175 »M dTTP, 25 »M Digoxigenin-11-2'-dUTP (Boehringer Mannheim), 50 mM KCl, 10 mM Tris-HCl (pH 8,3), 1,5 mM MgCl₂, 0,01 % Gelatine, 2,5 U Thermus aquaticus DNA-Polymerase. Der Reaktionsansatz wird mit 100 »l Mineralöl überschichtet und in einem Thermo-Cycler (Perkin Elmer) 30 Zyklen inkubiert: 30 sec 92°C, 30 sec 50°C und 1 min 70°C.

Durch den Amplifikationsschritt wird ein 523 bp großes DNA-Fragment erzeugt (HBV-PCR-Primer 1: Position 1937-1960, HBV-PCR-Primer 2: Position 2434-2460 im HBV-Genom).

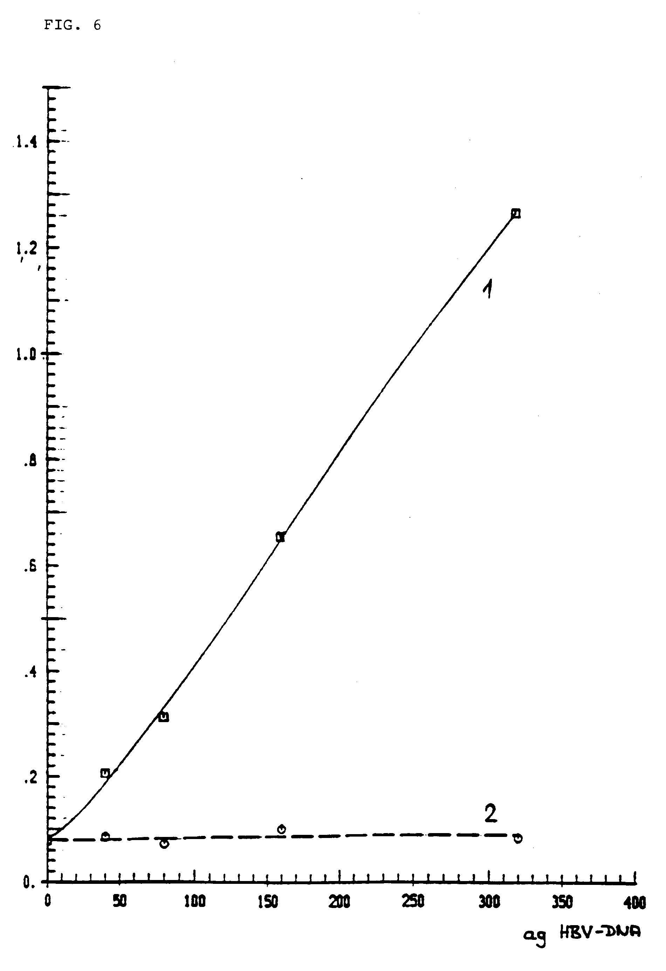

Der spezifische Nachweis der PCR-Produkte erfolgt in einem Zwei-Komponenten-Testsystem an der Festphase. Zunächst werden 20 »l des Amplifikationsansatzes mit 20 »l TE-Puffer (10 mM Tris-HCl (pH 7,5) 1 mM EDTA) und 10 »l 0,5 N NaOH versetzt und 10 min bei Raumtemperatur inkubiert. Die Neutralisation erfolgt durch Zugabe von 450 »l angesäuerter Hybridisierungslösung (50 mM Na-Phosphatpuffer, 0,05 N HCl, 0,75 M NaCl, 0,075 M Na-Citrat, 0,05 % RSA, pH 5,4), der 100 ng/ml eines Biotin-markierten Oligonukleotids zugesetzt ist, das einer Region des amplifizierten DNA-Bereiches entspricht (HBV-Biotin-Oligonukleotid, 30mer, 2fach Biotin-markiert, Position 2327-2356 im HBV-Genom, Bio-Oli 25, hergestellt durch Festphasensynthese, biotinmarkiert wie unter Beispiel 2 beschrieben). 200 »l dieses Hybridisierungsansatzes werden in einer Kavität einer mit Streptavidin beschichteten Mikrotiterplatte 3 h bei 37°C unter Schütteln inkubiert. Nach der Hybridisierungs- und Wandbindungsreaktion wird die Lösung abgesaugt und 2 x 10 min mit 0,3 M NaCl, 0,03 M Na-Citrat, 0,2 % SDS bei 37°C und 1 x kurz mit 0,9 % NaCl bei Raumtemperatur gewaschen. Nach Zugabe von 200 mU/ml anti-Digoxigenin-Antikörper-Meerrettichperoxidase-Konjugat in 100 mM Tris-HCl (pH 7,5), 0,9 % NaCl, 1 % RSA wird 30 min bei 37°C unter Schütteln inkubiert. Ungebundenes Konjugat wird durch 3maliges Waschen mit 0,9 % NaCl bei Raumtemperatur entfernt. Nach 30minütiger Inkubation mit 1,9 mM ABTS^{R} (2,2'-Azino-di-[3-ethyl-benzthiazolin-sulfonsäure (6)]-diammoniumsalz) bei 37°C wird die Extinktion bei 405 nm mittels eines ELISA-Readers gemessen. Die Ergebnisse sind in Figur 6 dargestellt.

## Patentansprüche

1. Verfahren zum spezifischen Nachweis einer Nukleinsäure in einer Probe, welches folgende Schritte umfaßt:
a) Reaktion der Probe mit einem oder mehreren mit einer nachweisbaren Gruppe markierten Mononukleosidtriphosphaten und einem oder mehreren Enzymen, welche die Herstellung einer markierten Nukleinsäure B katalysieren, die dieses Nukleotid enthält,
b) nichtthermische Denaturierung der Reaktionsmischung,
c) Hybridisierung der Nukleinsäure B mit einer Nukleinsäuresonde C, welche mindestens eine immobilisierbare Gruppe enthält und welche hinreichend komplementär zu der Nukleinsäure B ist, und gleichzeitige Immobilisierung der Nukleinsäuresonde C an eine feste Phase, welche die immobilisierbare Nukleinsäuresonde C spezifisch binden kann,
d) Trennung der flüssigen von der festen Phase, und
e) Nachweis des aus markierter Nukleinsäure B und Nukleinsäuresonde C gebildeten Nukleinsäurehybrids D, indem die an die feste Phase gebundene nachweisbare Gruppe nachgewiesen wird, wobei sich die immobilisierbare Gruppe von der nachzuweisbaren Gruppe unterscheidet.

2. Verfahren nach Anspruch 1, dadurch gekennzeichnet, daß Reaktion a) in Anwesenheit eines spezifischen Starters abläuft.

3. Verfahren gemäß Anspruch 1 oder 2, dadurch gekennzeichnet, daß das Enzym bzw. der Enzymkomplex die Polymerisation von Nukleosidtriphosphaten zu einer zu der Nukleinsäure A im wesentlichen komplementären Nukleinsäure B katalysiert.

4. Verfahren gemäß Anspruch 3, dadurch gekennzeichnet, daß die Nukleosidtriphosphate Ribonukleosidtriphosphate umfassen.

5. Verfahren gemäß Anspruch 2, dadurch gekennzeichnet, daß in Reaktion a) als spezifischer Starter ein Primer P1 benutzt wird, der zu einem Teil der Nukleinsäure A im wesentlichen komplementär ist und von dem Enzym unter Einbau des Mononukleosidtriphosphats zu einer zu der Nukleinsäure A im wesentlichen komplementären Nukleinsäure B verlängert wird.

6. Verfahren gemäß Anspruch 5, dadurch gekennzeichnet, daß außerdem ein Primer P2 benutzt wird, der zu einem Teil der Nukleinsäure B im wesentlichen komplementär ist.

7. Verfahren gemäß einem der Ansprüche 3 oder 5, dadurch gekennzeichnet, daß die Nukleosidtriphosphate Desoxyribonukleosidtriphosphate umfassen.

8. Verfahren nach einem der Ansprüche 1 bis 3, dadurch gekennzeichnet, daß die nachzuweisende Nukleinsäure einzelsträngig ist oder gemacht wird, der Probe dann mindestens ein Adaptor pro nachzuweisendem Einzelstrang zugegeben wird, welcher eine zu einem Teil der nachzuweisenden Nukleinsäure im wesentlichen komplementäre Nukleotidsequenz und eine Replikationsinitiationsregion enthält, der Adaptor um eine zu der Nukleinsäure komplementäre Sequenz verlängert wird, und die so gebildete Nukleinsäure als template-Nukleinsäure in Reaktion a) eingesetzt wird.

9. Verfahren nach einer der Ansprüche 1 bis 3, dadurch gekennzeichnet, daß die nachzuweisende Nukleinsäure einzelsträngig ist oder gemacht wird, der Probe dann mindestens ein Primer pro nachzuweisendem Einzelstrang zugesetzt wird, welcher eine zu einem Teil der nachzuweisenden Nukleinsäure im wesentlichen komplementäre Nukleotidsequenz und eine Transkriptionsinitiationssequenz enthält, der Primer um eine zu der nachzuweisenden Nukleinsäure komplementäre Nukleotidsequenz verlängert und die so gebildete Nukleinsäure als template-Nukleinsäure in Reaktion a) eingesetzt wird.

10. Verfahren gemäß einem der vorangehenden Ansprüche, dadurch gekennzeichnet, daß die in Schritt a) gebildete Nukleinsäure B als template-Nukleinsäure erneut in Reaktion a) eingesetzt wird.

11. Verfahren gemäß einem der vorangehenden Ansprüche, dadurch gekennzeichnet, daß Reaktion a) mehrmals hintereinander ausgeführt wird, wobei jeweils die Produkte der Reaktion wieder als Ausgangsstoffe der erneuten Reaktion dienen.

12. Verfahren gemäß Anspruch 10 oder 11, dadurch gekennzeichnet, daß in Reaktion a) ein Nukleinsäurehybrid aus Nukleinsäure A und Nukleinsäure B gebildet wird.

13. Verfahren gemäß einem der vorangegangenen Ansprüche, dadurch gekennzeichnet, daß die Denaturierung bei alkalischem pH stattfindet.

14. Verfahren gemäß einem der vorangehenden Ansprüche, dadurch gekennzeichnet, daß die Denaturierung durch Zugabe chaotroper Salze vorgenommen wird.

15. Verfahren gemäß Anspruch 13 oder 14, dadurch gekennzeichnet, daß die Denaturierung in einem Temperaturbereich zwischen 17°C und 50°C stattfindet.

16. Verfahren gemäß einem der Ansprüche 13, 14 oder 15, dadurch gekennzeichnet, daß das gesamte Nachweisverfahren in einem Temperaturbereich zwischen 17°C und 50°C stattfindet.

17. Verfahren gemäß Anspruch 16, dadurch gekennzeichnet, daß das gesamte Nachweisverfahren bei einer einzigen Temperatur stattfindet.

18. Verfahren gemäß Anspruch 11, dadurch gekennzeichnet, daß die Nukleinsäuresonde in einer Lösung mit einem pH zwischen 3.0 und 6.5 zugegeben wird.

19. Verfahren gemäß Anspruch 18, dadurch gekennzeichnet, daß die Lösung außerdem alle für die Durchführung der Hybridisierung erforderlichen Reagenzien enthält.

20. Verfahren gemäß Anspruch 18 oder 19, dadurch gekennzeichnet, daß die Nukleinsäuresonde C eine einzelsträngige Nukleinsäure C1 ist, deren Gegenstrang nicht in der Lösung vorhanden ist.

21. Verfahren gemäß Anspruch 13, dadurch gekennzeichnet, daß die Nukleinsäuresonde C in einer Lösung mit einem pH zwischen 10 und 14 zugegeben wird und anschließend mit der Hybridisierungslösung in der Mischung ein pH zwischen 5.0 und 8.5 eingestellt wird.

22. Verfahren gemäß Anspruch 13, dadurch gekennzeichnet, daß zunächst eine Lösung der Sonde mit der Probe vereinigt, dann ein pH zwischen 10 und 14 eingestellt wird und anschließend in der Mischung ein pH zwischen 5.0 und 8.5 eingestellt wird.

23. Verfahren nach Anspruch 6, dadurch gekennzeichnet, daß die durch Verlängerung der Primer P1 und P2 gebildeten Nukleinsäuren nach Strangtrennung erneut mit P1 und P2 umgesetzt werden, wobei die mit dem einen Primer neu gebildeten Stränge jeweils als template für die Verlängerung des anderen Primers dienen.

## Claims

1. Process for the specific detection of a nucleic acid in a sample which includes the following steps:
a) reaction of the sample with one or more mononucleoside triphosphates labelled with a detectable group and one or more enzymes which catalyse the production of a labelled nucleic acid B which contains this nucleotide,
b) non-thermal denaturing of the reaction mixture,
c) hybridisation of the nucleic axid B with a nucleic acid probe C which contains at least one immobilisable group and which is sufficiently complementary to the nucleic acid B and simultaneous immobilisation of the nucleic acid probe C on a solid phase which can specifically bind the immobilisable nucleic acid probe C,
d) separation of the liquid from the solid phase and
e ) detection of the nucleic acid hybrid D formed from labelled nucleic acid B and nucleic acid probe C in that the detectable group bound to the solid phase is detected, whereby the immobilisable group differs from the group to be detected.

2. Process according to claim 1, characterised in that reaction a) takes place in the presence of a specific starter.

3. Process according to claim 1 or 2, characterised in that the enzyme of the enzyme complex catalyses the polymerisation of nucleoside triphosphates to a nucleic acid B substantially complementary to the nucleic acid A.

4. Process according to claim 3, characterised in that the nucleoside triphosphates include ribonucleoside triphosphates.

5. Process according to claim 2, characterised in that, in reaction a), a primer P1 is used as specific starter which is substantially complementary to a part of the nucleic acid A and is elongated by the enzyme with incorporation of the mononucleoside triphosphate to give a nucleic acid B substantially complementary to the nucleic acid A.

6. Process according to claim 5, characterised in that furthermore a primer P2 is used which is substantially complementary to a part of the nucleic acid B.

7. Process according to one of claims 3 or 5, characterised in that the nucleotide triphosphates include desoxyribonucleoside triphosphates.

8. Process according to one of claims 1 to 3, characterised in that the nucleic acid to be detected is or is made single-stranded, to the sample is then added at least one adaptor per single strand to be detected which contains a nucleotide sequence substantially complementary to a part of the nucleic acid to be detected and a replication initiation region, the adaptor is elongated by a sequence complementary to the nucleic acid and the so-formed nucleic acid is used as template nucleic acid in reaction a).

9. Process according to one of claims 1 to 3, characterised in that the nucleic acid to be detected is or is made single-stranded, to the sample is then added at least one primer per single strand to be detected which contains a nucleotide sequence substantially complementary to a part of the nucleic acid to be detected and a transcription initiation sequence, the primer is elongated by a nucleotide sequence complementary to the nucleic acid to be detected and the so-formed nucleic acid is used as template nucleic acid in reaction a).

10. Process according to one of the preceding claims, characterised in that the nucleic acid B formed in step a) is again used as template nucleic acid in reaction a).

11. Process according to one of the preceding claims, characterised in that reaction a) is carried out several times in succession, whereby, in each case, the products of the reaction again serve as starting materials of the renewed reaction.

12. Process according to claim 10 or 11, characterised in that, in reaction a), a nucleic acid hybrid is formed from nucleic acid A and nucleic acid B.

13. Process according to one of the preceding claims, characterised in that the denaturing takes place at alkaline pH.

14. Process according to one of the preceding claims, characterised in that the denaturing is carried out by addition of chaotropic salts.

15. Process according to claim 13 or 14, characterised in that the denaturing takes place in a temperature range between 17°C and 50°C.

16. Process according to one of claims 13, 14 or 15, characterised in that the whole detection process takes place in a temperature range between 17°C and 50°C.

17. Process according to claim 16, characterised in that the whole detection process takes place at a single temperature.

18. Process according to claim 11, characterised in that the nucleic acid probe is added in a solution with a pH between 3.0 and 6.5.

19. Process according to claim 18, characterised in that the solution also contains all reagents necessary for the carrying out of the hybridisation.

20. Process according to claim 18 or 19, characterised in that the nucleic acid probe C is a single-stranded nucleic acid C1, the counter-strand of which is not present in the solution.

21. Process according to claim 13, characterised in that the nucleic acid probe C is added in a solution with a pH between 10 and 14 and subsequently a pH between 5.0 and 8.5 is adjusted in the mixture with the hybridisation solution.

22. Process according to claim 13, characterised in that first a solution of the probe is combined with the sample, then a pH between 10 and 14 is adjusted and subsequently a pH between 5.0 and 8.5 is adjusted in the mixture.

23. Process according to claim 6, characterised in that the nucleic acids formed by elongation of the primers P1 and P2 are, after strand separation, again reacted with P1 and P2, whereby the strands newly formed with one primer serve in each case as template for the elongation of the other primer.

## Revendications

1. Procédé de détection spécifique d'un acide nucléique dans un échantillon, comprenant les étapes consistant à :
a) faire réagir un échantillon avec ou plusieurs mononucléosidetriphosphate(s), marqué(s) avec un groupe détectable, et une ou plusieurs enzyme(s) qui catalysent la préparation d'un acide nucléique B contenant ce nucléotide,
b) dénaturer le mélange réactionnel par un procédé non thermique,
c) hybrider l'acide nucléique B avec une sonde nucléique C, qui contient au moins un groupe immobilisable et qui est suffisamment complémentaire de l'acide nucléique B, et immobiliser dans le même temps la sonde nucléique C sur une phase solide, à laquelle la sonde nucléique C peut se lier de manière spécifique,
d) séparer les substances liquides de la phase solide, et
e) détecter l'hybride d'acide nucléique D formé à partir de l'acide nucléique a marqué et de la sonde nucléique C, par détection du groupe détectable fixé à la phase solide, le groupe immobilisable se différenciant du groupe détectable.

2. Procédé selon la revendication 1, caractérisé en ce que la réaction a) se déroule en présence d'un starter spécifique.

3. Procédé selon la revendication 1 ou 2, caractérisé en ce que l'enzyme ou le complexe d'enzyme catalyse la polymérisation de nucléosidetriphosphates en un acide nucléique B essentiellement complémentaire de l'acide nucléique A.

4. Procédé selon la revendication 3, caractérisé en ce que les nucléosidetriphosphates comprennent des ribonucléosidetriphosphates.

5. Procédé selon la revendication 2, caractérisé en ce que dans la réaction a), en tant que starter spécifique, on utilise une amorce P1 qui est essentiellement complémentaire d'une partie de l'acide nucléique A et qui est allongée par l'enzyme par incorporation du mononucléosidetriphosphate dans un acide nucléique B essentiellement complémentaire de l'acide nucléique A.

6. Procédé selon la revendication 5, caractérisé en ce que l'on utilise en outre une amorce P2 qui est essentiellement complémentaire d'une partie de l'acide nucléique B.

7. Procédé selon l'une quelconque des revendications 3 ou 5, caractérisé en ce que les nucléosidetriphosphates comprennent des désoxyribonucléosidetriphosphates.

8. Procédé selon l'une quelconque des revendications 1 à 3, caractérisé en ce que l'acide nucléique à détecter est monobrin ou est rendu monobrin, qu'on ajoute alors à l'échantillon, par monobrin à détecter, au moins un adaptateur, qui contient une séquence nucléotidique essentiellement complémentaire d'une partie de l'acide nucléique à détecter, et une région d'initiation de la réplication, que l'on allonge l'adaptateur d'une séquence complémentaire de l'acide nucléique et que l'on met en oeuvre l'acide nucléique ainsi formé en tant qu'acide nucléique matrice dans la réaction a).

9. Procédé selon l'une quelconque des revendications 1 à 3, caractérisé en ce que l'acide nucléique à détecter est monobrin ou est rendu monobrin, que l'on ajoute alors à l'échantillon, par monobrin à détecter, au moins un adaptateur, qui contient une séquence nucléotidique essentiellement complémentaire d'une partie de l'acide nucléique à détecter et une séquence d'initiation de la transcription, que l'on allonge l'amorce d'une séquence nucléotidique complémentaire de l'acide nucléique à détecter et que l'on met en oeuvre l'acide nucléique ainsi formé en tant qu'acide nucléique matrice dans la réaction a).

10. Procédé selon l'une quelconque des revendications précédentes, caractérisé en ce que l'acide nucléique B formé dans l'étape a) est utilisé à nouveau dans la réaction a) en tant qu'acide nucléique matrice.

11. Procédé selon l'une quelconque des revendications précédentes, caractérisé en ce que l'on effectue la réaction a) plusieurs fois de suite, les produits de la réaction servant chaque fois comme produits de départ de la réaction répétée.

12. Procédé selon la revendication 10 ou 11, caractérisé en ce que dans la réaction a), il se forme un acide nucléique hybridé à partir de l'acide nucléique A et de l'acide nucléique B.

13. Procédé selon l'une quelconque des revendications précédentes, caractérisé en ce que la dénaturation a lieu à un pH alcalin.

14. Procédé selon l'une quelconque des revendications précédentes, caractérisé en ce que la dénaturation est réalisée par addition de sels chaotropes.

15. Procédé selon la revendication 13 ou 14, caractérisé en ce que la dénaturation est réalisée dans une gamme de températures comprise entre 17°C et 50°C.

16. Procédé selon la revendication 13, 14 ou 15, caractérisé en ce que toute la réaction de détection est réalisée dans une gamme de températures comprise entre 17°C et 50°C.

17. Procédé selon la revendication 16, caractérisé en ce que toute la réaction de détection est réalisée à une seule et même température.

18. Procédé selon la revendication 11, caractérisé en ce que la sonde nucléique est ajoutée dans une solution ayant un pH compris entre 3,0 et 6,5.

19. Procédé selon la revendication 18, caractérisé en ce que la solution contient en outre tous les réactifs nécessaires à la réalisation de l'hybridation.

20. Procédé selon la revendication 18 ou 19, caractérisé en ce que la sonde nucléique C est un acide nucléique monobrin C1, dont le brin opposé n'est pas présent dans la solution.

21. Procédé selon la revendication 13, caractérisé en ce que la sonde nucléique C est ajoutée dans une solution ayant un pH compris entre 10 et 14, et ensuite, avec la solution d'hybridation, le pH du mélange est ajusté entre 5,0 et 8,5.

22. Procédé selon la revendication 13, caractérisé en ce que d'abord, on réunit une solution de la sonde avec l'échantillon, le pH étant alors ajusté entre 10 et 14 et ensuite, on ajuste le pH du mélange entre 5,0 et 8,5.

23. Procédé selon la revendication 6, caractérisé en ce que les acides nucléiques formés par allongement des amorces P1 et P2 sont mis à réagir à nouveau, après séparation des brins, avec P1 et P2, les brins nouvellement formés avec l'une des amorces servant chaque fois à allonger l'autre amorce.
